# EUROPEAN PATENT SPECIFICATION

(11) **EP 0 277 419 B1**
(45) Date of publication and mention of the grant of the patent: **18.06.1997**
(21) Application number: 87310487.1
(22) Date of filing: 27.11.1987
(51) Int. Cl.: C07K 14/655, A61K 38/08

(54) **Therapeutic somatostatin analogues**
Therapeutische Somatostatin-Analoge
Analogues thérapeutiques de la somatostatine

(30) Priority: 03.02.1987 US 10349
(43) Date of publication of application: 10.08.1988
(73) Proprietor: THE ADMINISTRATORS OF THE TULANE UNIVERSITY EDUCATIONAL FUND, New Orleans, Louisiana 70112-2699 (US)
(72) Inventor: Coy, David H., New Orleans Louisiana 70115 (US); Murphy, William A., Covington Louisiana 70435 (US); Heiman, Mark L., New Orleans Louisiana 70122 (US)
(74) Representative: Sheard, Andrew Gregory

(56) References cited:
- EP-A- 0 214 872
- EP-A- 0 215 171
- US-A- 4 328 135
- US-A- 4 440 904
- CHEMICAL ABSTRACTS, vol. 111, 1989, page 78, abstract no. 50516d, Columbus,
- Ohio, US; D.H. COY et al.: "Receptor-selective somatostatin (SRIF) analogs", & CHEM. BIOL., PROC. AM. PEPT. SYMP. 10TH 1987 (PUB. 1988), 462-4
- Biochem Biophys Res Commun 132 (1985) 922-938
- Chem Biol Proc Am Pept Symp 462-4

## Description

This invention relates to therapeutic peptides.

A number of somatostatin analogues exhibiting GH-release-inhibiting activity have been described in the literature, including analogs containing fewer than the naturally occurring fourteen amino acids. For example, Coy et al. U.S. Patent No. 4,485,101, hereby incorporated by reference, describes dodecapeptides having an N-terminal acetyl group, a C-terminal NH₂, D-Trp at position 6, and p-Cl-Phe at position 4. (Herein, when no designation of configuration is given, the L-isomer is intended.)

In a first aspect the invention features an octapeptide of the formula: H-A¹-Cys-Tyr-D-Trp-Lys-α-aminobutyric acid-Cys-A², wherein:
A¹ is D-β-Nal,or D-Phe; and A² is Thr-NH₂, or β-Nal-NH₂ or a pharmaceutically acceptable salt thereof.

Preferred compounds of the invention include H-D-Phe-Cys-Tyr-D-Trp-Lys-α-Aminobutyric acid-Cys-Thr-NH₂ and H-D-β-Nal-Cys-Tyr-D-Trp-Lys-α-aminobutyric acid-Cys-Thr-NH₂.

In other preferred embodiments, according to the invention is the therapeutic compound and a pharmaceutically acceptable carrier substance, e.g. magnesium carbonate, lactose, or a phospholipid with which the therapeutic compound can form a micelle, and which together form a therapeutic composition,
e.g. a pill, tablet, capsule, or liquid for oral administration to a human patient, a spreadable cream, gel, lotion, or ointment for application to the skin of a human patient in need of the compound, a liquid capable of being administered nasally as drops or spray, or a liquid capable of intravenous, parenteral, subcutaneous, or intraperitoneal administration. The pill, tablet or capsule can be coated with a substance capable of protecting the composition from the gastric acid in the patient's stomach for a period of time sufficient to allow the composition to pass undisintegrated into the patient's small intestine. The therapeutic composition can also be in the form of a biodegradable sustained release formulation for intramuscular administration. For maximum efficacy, zero order release is desired. Zero order release can be obtained using an implantable or external pump,
e.g., Infusoid ^{TM} pump, to administer the therapeutic composition.

The compounds of the invention are active in inhibiting the secretion of GH, insulin, and glucagon. Further, the aromatic lipophilic N-terminal end can provide long-lasting in vivo activity.

Other features and advantages of the invention will be apparent from the following description of the preferred embodiments thereof, and from the claims.

### Description of the Preferred Embodiments

### Structure

The compounds of the invention have the general formula recited in claim 1 and above.
They are all octapeptide analogues of somatostatin which have Cys at position 2; Tyr at position 3; D-Trp. at position 4; Lys at position 5; Abu at position 6 (where Abu is α-Aminobutyric Acid); and Cys at position 7. It has been found that D-β-Nal at position 1 particularly enhances activity.

The compounds can be provided in the form of pharmaceutically acceptable salts. Examples of preferred salts are those with therapeutically acceptable organic acids, e.g., acetic, lactic, maleic, citric, malic, ascorbic, succinic, benzoic, salicylic, methanesulphonic, toluenesulphonic, or pamoic acid, as well as polymeric acids such as tannic acid or carboxymethyl cellulose, and salts with inorganic acids such as the hydrohalic acids, e.g., hydrochloric acid, sulfuric acid, or phosphoric acid.

### Synthesis

The synthesis of one octapeptide follows. Other octapeptides of the invention can be prepared by making appropriate modifications, within the ability of someone of ordinary skill in this field, of the following synthetic method.

The first step in the preparation of D-β-naphthylalanine-Cys-Tyr-D-Trp-Lys-Abu-Cys-Thr- NH₂ was the preparation of the intermediate tert-butyloxycarbonyl-D-β-naphthylalanine-S-methylbenzyl-Cys-Tyr-D-Trp-N^{ε}-benzyloxycarbonyl-Lys-Abu-S-methylbenzyl-Cys-O-benzyl-Thr-benzyhydrylaminine resin, as follows.

Benzhydrylamine-polystyrene resin (Vega Biochemicals, Inc.) in the chloride ion form was placed in the reaction vessel of a Beckman 990B peptide synthesizer programmed to perform the following reaction cycle: (a) methylene chloride; (b) 33% trifluoroacetic acid in methylene chloride (2 times for 1 and 25 min each); (c) methylene chloride; (d) ethanol; (e) methylene chloride; (f) 10% triethylamine in chloroform.

The neutralized resin was stirred with Boc-O-benzyl-threonine and diisopropylcarbodiimide (1.5 mmole each) in methylene chloride for 1 h and the resulting amino acid resin was then cycled through steps (a) to (g) in the above wash program. The following amino acids (1.5 mmole) were then coupled successively by the same procedure: Boc-S-methylbenzyl-Cys, Boc-N-α-benzyloxycarbonyl-Abu, Boc-Ne-benzyloxycarbonyl-lysine, Boc-D-Trp, Boc-Tyr, Boc-S-methylbenzyl-Cys, Boc-D-β-naphthylalanine.

The resin was washed and dried and then mixed with anisole (4 ml) and anhydrous hydrogen fluoride (36 ml) at 0°C and stirred for 45 min. (one can also use thioanisole, trifluoroacetic acid, and trifluoromethane sulfonic acid at a ratio of 1:90:9, for 6h). Excess hydrogen fluoride was evaporated rapidly under a stream of dry nitrogen and free peptide precipitated and washed with ether. The crude peptide was then dissolved in 800 ml of 90% acetic acid to which was added I₂ in methanol until a permanent brown color was present. The solution was then stirred for 1 h before removing the solvent in vacuo. The resulting oil was dissolved in a minimum volume of 50% acetic acid and eluted on a column (2.5 X 100 mm) of Sephadex G-25. Fractions containing a major component by uv absorption and thin layer chromatography were then pooled, evaporated to a small volume, and applied to a column (2.5 X 50 cm) of Whatman LRP-1 octadecylsilane (15-20 uM).

The column was eluted with a linear gradient of 10-50% acetonitrile in 0.1% trifluoroacetic acid in water. Fractions were examined by thin layer chromatography and analytical high performance liquid chromatography and pooled to give maximum purity and if desired, a different salt prepared, e.g., acetate or phosphate. Repeated lyophilization of the solution from water gave 170 mg of the product as a white, fluffy powder.

The product was found to be homogeneous by Hplc and Tlc. Amino acid analysis of an acid hydrolysate confirmed the composition of the octapeptide.

### Use

When administered to mammals, particularly humans, (e.g. orally, topically, intravenously, parenterally in a sustained release, biodegradable form, nasally, or by suppository), the compounds can be effective to inhibit GH release as well as to inhibit insulin, glucagon, and pancreatic exocrine secretion, and to therapeutically affect the central nervous system.

The compounds can be administered to a mammal, e.g. a human, in the dosages used for somatostatin or, because of their greater potency, in smaller dosages. The compounds of the invention can be used for the treatment of cancer, particularly growth hormone-dependent cancer (e.g., bone, cartilage, pancreas (endocrine and exocrine), prostate, or breast), acromegaly and related hypersecretory endocrine states, or of bleeding ulcers in emergency patients and in those suffering from pancreatitis or diarrhea. The compounds can also be used in the management of diabetes and to protect the liver of patients suffering from cirrhosis or hepatitis. The compounds can also be used to treat Alzheimer's disease, as analgesics to treat pain by acting specifically on certain opiate receptors, and as gastric cytoprotective compounds for ulcer therapy. The compounds can also be used to treat certain types of mushroom poisoning.

The compounds can also be used to treat diabetes-related retinopathy. The anti-cancer activity of the compounds may be related to their ability to antagonize cancer-related growth factors such as epidermal growth factor.

The compounds can be administered to a mammal, e.g., a human, in a dosage of 0.01 to 1000 mcg/kg/day, preferably 0.1 to 100 mcg/kg/day.

## Claims (Claims for the following Contracting State(s): AT, BE, CH, DE, FR, GB, IT, LI, LU, NL, SE)

1. An octapeptide of the formula: H-A¹-Cys-Tyr-D-Trp-Lys-α-aminobutyric acid-Cys-A², wherein:
A¹ is D-β-Nal or D-Phe;
A² is Thr-NH₂, or β-Nal-NH₂;
or a pharmaceutically acceptable salt thereof.

2. An octapeptide according to claim 1 wherein A¹ is D-β-Nal.

3. An octapeptide of the formula:
(i) H-D-Phe-Cys-Tyr-D-Trp-Lys-α-aminobutyric acid-Cys-Thr-NH₂,
(ii) H-D-β-Nal-Cys-Tyr-D-Trp-Lys-α-aminobutyric acid-Cys-Thr-NH₂;
or a pharmaceutically acceptable salt thereof.

4. A therapeutic composition comprising a compound as claimed in claim 1, 2 or 3 together with a pharmaceutically acceptable carrier substance.

5. A composition as claimed in claim 4 wherein the composition is in the form of a liquid, pill, tablet, or capsule for oral administration.

6. A composition as claimed in claim 4 wherein the composition is coated with a substance capable of protecting the composition from the gastric acid in the stomach of a human patient for a period of time sufficient to allow the composition to pass undisintegrated into the small intestine of the human patient.

7. A composition as claimed in claim 4 wherein:
(a) the composition is in the form of a cream, gel, spray, or ointment for application to the skin of a human patient in need of the compound;
(b) the composition is in the form of a liquid capable of being administered nasally as drops or spray to a human patient in need of the compound;
(c) the composition is in the form of a liquid for intravenous, subcutaneous, parenteral, or intraperitioneal administration to a human patient in need of the compound; or
(d) the composition is in the form of a biodegradable sustained release composition for intramuscular administration to a human patient in need of said compound.

8. An octapeptide as claimed in claim 1, 2 or 3 for use in medicine.

9. The use of an octapeptide as claimed in claim 1, 2 or 3 in the preparation of an agent for reducing growth hormone, insulin, glucagon and/or pancreatic exocrine secretion.

10. A process for the preparation of an octapeptide as claimed in claim 1, 2 or 3, the process comprising coupling together successive amino acids by peptide bond formation.

## Claims (Claims for the following Contracting State(s): ES, GR)

1. A process for the preparation of an octapeptide of the formula: H-A¹-Cys-Tyr-D-Trp-Lys-α-aminobutyric acid-Cys-A², wherein:
A¹ is D-β-Nal or D-Phe;
A² is Thr-NH₂, or β-Nal-NH₂;
or a pharmaceutically acceptable salt thereof.

2. A process according to claim 1 wherein A¹ is D-β-Nal.

3. A process for the preparation of an octapeptide of the formula:
(i) H-D-Phe-Cys-Tyr-D-Trp-Lys-α-aminobutyric acid-Cys-Thr-NH₂;
(ii) H-D-β-Nal-Cys-Tyr-D-Trp-Lys-α-aminobutyric acid-Cys-Thr-NH₂;
or a pharmaceutically acceptable salt thereof comprising coupling successive amino acids together by peptide bond formation.

4. A process for the preparation of a therapeutic composition, the process comprising admixing an octapeptide as defined in claim 1, 2 or 3 with a pharmaceutically acceptable carrier therefor.

5. A process as claimed in claim 4 wherein the composition is in the form of a liquid, pill, tablet, or capsule for oral administration to a human patient in need of the compound.

6. A process as claimed in claim 4 wherein the composition is coated with a substance capable of protecting the composition from the gastric acid in the stomach of the human patient for a period of time sufficient to allow the composition to pass undisintegrated into the small intestine of the human patient.

7. A process as claimed in claim 4 wherein the composition is in the form of a cream, gel, spray, or ointment for application to the skin of a human patient in need of the compound or the composition is in the form of a liquid capable of being administered nasally as drops or spray to a human patient in need of the compound.

8. A process as claimed in claim 4 wherein the composition is in the form of a liquid for intravenous, subcutaneous, parenteral, or intraperitioneal administration to a human patient in need of the compound.

9. A process as claimed in claim 4 wherein the composition is in the form of a biodegradable sustained release composition for intramuscular administration to a human patient in need of the compound.

10. The use of an octapeptide as defined in claim 1, 2 or 3 in the preparation of an agent for reducing growth hormone, insulin, glucagon and/or pancreatic exocrine secretion.

## Patentansprüche (Patentansprüche für folgende(n) Vertragsstaat(en): AT, BE, CH, DE, FR, GB, IT, LI, LU, NL, SE)

1. Ein Octapeptid der Formel: H- A¹-Cys-Tyr-D-Trp-Lys-α-Aminobuttersäure-Cys-A², in welcher:
A¹ D-β-Nal oder D-Phe;
A² Thr-NH₂ oder β-Nal-NH₂;
oder ein phamazeutisch annehmbares Salz davon bedeutet.

2. Ein Octapeptid nach Anspruch 1, in welchem A¹ D-β-Nal bedeutet.

3. Ein Octapeptid der Formel:
(i) H-D-Phe-Cys-Tyr-D-Trp-Lys-α-Aminobuttersäure-Cys-Thr-NH₂;
(ii) H-D-β-Nal-Cys-Tyr-D-Trp-Lys-α-Aminobuttersäure-Cys-Thr-NH₂; oder ein pharmazeutisch annehmbares Salz davon.

4. Therapeutische Zusammensetzung, welche eine Verbindung wie in den Ansprüchen 1, 2 oder 3 beansprucht zusammen mit einem pharmazeutisch annehmbaren Träger enthält.

5. Zusammensetzung nach Anspruch 4, wobei die Zusammensetzung in Form einer Flüssigkeit, Pille, Tablette oder Kapsel zur oralen Verabreichung vorliegt.

6. Zusammensetzung nach Anspruch 4, wobei die Zusammensetzung mit einer Substanz überzogen ist, die die Zusammensetzung vor der Magensäure in dem Magen eines menschlichen Patienten für eine Zeit schützt, die ausreicht, um der Zusammensetzung zu ermöglichen, unzersetzt in den Dünndarm des menschlichen Patienten zu gelangen.

7. Zusammensetzung nach Anspruch 4, wobei:
(a) die Zusammensetzung in Form einer Creme, eines Gels, Sprays, oder einer Salbe zur Applikation auf die Haut eines die Zusammensetzung benötigenden menschlichen Patienten vorliegt;
(b) die Zusammensetzung in Form einer Flüssigkeit vorliegt, die geeignet ist, nasal als Tropfen oder Spray an einen die Zusammensetzung benötigenden Patienten verabreicht zu werden;
(c) die Zusammensetzung in Form einer Flüssigkeit für eine intravenöse, subcutane, parenterale oder intraperitoneale Verabreichung an einen die Zusammensetzung benötigenden menschlichen Patienten vorliegt;
(d) die Zusammensetzung in Form einer biologisch abbaubaren Zusammensetzung mit verzögerter Freisetzung zur intramuskulären Verabreichung an einen diese Zusammensetzung benötigenden menschlichen Patienten vorliegt.

8. Ein Octapeptid wie in Anspruch 1, 2 oder 3 beansprucht zur Verwendung in der Medizin.

9. Verwendung eines Octapeptids wie in Anspruch 1, 2 oder 3 beansprucht bei der Herstellung eines Mittels zur Verringerung der Sekretion von Wachstumshormon, Insulin, Glucagon und/oder pancreatischem Exocrin.

10. Verfahren zur Herstellung eines Octapeptids wie in Anspruch 1, 2 oder 3 beansprucht, wobei das Verfahren das Aneinanderkoppeln aufeinanderfolgender Aminosäuren durch Bildung von Peptidbindungen umfaßt.

## Patentansprüche (Patentansprüche für folgende(n) Vertragsstaat(en): ES, GR)

1. Verfahren zur Herstellung eines Octapeptids der Formel: H- A¹-Cys-Tyr-D-Trp-Lys-α-Aminobuttersäure-Cys-A², in welcher:
A¹ D-β-Nal oder D-Phe;
A² Thr-NH₂ oder β-Nal-NH₂;
oder ein phamazeutisch annehmbares Salz davon bedeutet.

2. Verfahren nach Anspruch 1, wobei A¹ D-β-Nal bedeutet.

3. Verfahren zur Herstellung eines Octapeptids der Formel:
(i) H-D-Phe-Cys-Tyr-D-Trp-Lys-α-Aminobuttersäure-Cys-Thr-NH₂;
(ii) H-D-β-Nal-Cys-Tyr-D-Trp-Lys-α-Aminobuttersäure-Cys-Thr-NH₂;
oder ein pharmazeutisch annehmbares Salz davon, wobei das Verfahren das Aneinanderkoppeln aufeinanderfolgender Aminosäuren durch Bildung von Peptidbindungen umfaßt.

4. Verfahren zur Herstellung einer therapeutischen Zusammensetzung, wobei das Verfahren das Mischen eines Octapeptids wie in Anspruch 1, 2 oder 3 beansprucht mit einem pharmazeutisch annehmbaren Träger dafür umfaßt.

5. Verfahren nach Anspruch 4, bei welchem die Zusammensetzung in Form einer Flüssigkeit, Pille, Tablette oder Kapsel zur oralen Verabreichung an einen die Zusammensetzung benötigenden menschlichen Patienten vorliegt.

6. Verfahren nach Anspruch 4, bei welchem die Zusammensetzung mit einer Substanz überzogen ist, die die Zusammensetzung vor der Magensäure in dem Magen eines menschlichen Patienten für eine Zeit schützt, die ausreicht, um der Zusammensetzung zu ermöglichen, unzersetzt in den Dünndarm des menschlichen Patienten zu gelangen.

7. Verfahren nach Anspruch 4, bei welchem die Zusammensetzung in Form einer Creme, eines Gels, Sprays, oder einer Salbe zur Applikation auf die Haut eines die Zusammensetzung benötigenden menschlichen Patienten vorliegt, oder die Zusammensetzung in Form einer Flüssigkeit vorliegt, die geeignet ist, nasal als Tropfen oder Spray an einen die Zusammensetzung benötigenden Patienten verabreicht zu werden.

8. Verfahren nach Anspruch 4, bei welchem die Zusammensetzung in Form einer Flüssigkeit für eine intravenöse, subcutane, parenterale oder intraperitoneale Verabreichung an einen die Zusammensetzung benötigenden menschlichen Patienten vorliegt.

9. Verfahren nach Anspruch 4, bei welchem die Zusammensetzung in Form einer biologisch abbaubaren Zusammensetzung mit verzögerter Freisetzung zur intramuskulären Verabreichung an einen diese Zusammensetzung benötigenden menschlichen Patienten vorliegt.

10. Verwendung eines Octapeptids wie in Anspruch 1, 2 oder 3 definiert bei der Herstellung eines Mittels zur Verringerung der Sekretion von Wachstumshormon, Insulin, Glucagon und/oder pancreatischem Exocrin.

## Revendications (Revendications pour l'(les) Etat(s) contractant(s) suivant(s): AT, BE, CH, DE, FR, GB, IT, LI, LU, NL, SE)

1. Octapeptide de formule : H-A¹-Cys-Tyr-D-Trp-Lys-α-acide aminobutyrique-Cys-A², où :
A¹ est D-β-Nal ou D-Phe;
A² est Thr-NH₂ ou β-Nal-NH₂;
ou un de ses sels pharmaceutiquement acceptables.

2. Octapeptide suivant la revendication 1, où A¹ est D-β-Nal.

3. Octapeptide de formule :
(i) H-D-Phe-Cys-Tyr-D-Trp-Lys-α-acide aminobutyrique-Cys-Thr-NH₂;
(ii) H-D-β-Nal-Cys-Tyr-D-Trp-Lys-α-acide aminobutyrique-Cys-Thr-NH₂;
ou un de ses sels pharmaceutiquement acceptables.

4. Composition thérapeutique comprenant un composé suivant la revendication 1, 2 ou 3, ainsi qu'une substance vectrice pharmaceutiquement acceptable.

5. Composition suivant la revendication 4, où la composition est sous la forme d'un liquide, d'une pilule, d'une comprimé ou d'une capsule, pour l'administration orale.

6. Composition suivant la revendication 4, où la composition est enrobée d'une substance capable de protéger la composition contre l'acide gastrique, dans l'estomac d'un patient humain, durant une période de temps suffisante pour permettre à la composition de passer, sans être désintégrée, dans l'intestin grêle du patient humain.

7. Composition suivant la revendication 4, où :
(a) la composition est sous la forme d'une crème, d'un gel, d'un spray ou d'une pommade, pour l'application sur la peau d'un patient humain nécessitant le composé;
(b) la composition est sous la forme d'un liquide pouvant être administré par voie nasale, sous forme de gouttes ou de spray, à un patient humain nécessitant le composé;
(c) la composition est sous la forme d'un liquide, pour l'administration intraveineuse, sous-cutanée, parentérale ou intrapéritonéale, à un patient humain nécessitant le composé;
(d) la composition est sous la forme d'une composition biodégradable à libération prolongée, pour l'administration intramusculaire à un patient humain nécessitant ce composé.

8. Octapeptide suivant la revendication 1, 2 ou 3, pour une utilisation en médecine.

9. Utilisation d'un octapeptide suivant les revendications 1, 2 ou 3 dans la préparation d'un agent de réduction de la sécrétion de l'hormone de croissance, de l'insuline, du glucagon et/ou de la sécrétion exocrine pancréatique.

10. Procédé de préparation d'un octapeptide suivant la revendication 1, 2 ou 3, le procédé comprenant le couplage l'un à l'autre d'acides aminés successifs par formation de liens peptidiques.

## Revendications (Revendications pour l'(les) Etat(s) contractant(s) suivant(s): ES, GR)

1. Procédé de préparation d'un octapeptide de formule : H-A¹-Cys-Tyr-D-Trp-Lys-α-acide aminobutyrique-Cys-A², où :
A¹ est D-β-Nal ou D-Phe;
A² est Thr-NH₂ ou β-Nal-NH₂;
ou un de ses sels pharmaceutiquement acceptables.

2. Procédé suivant la revendication 1, où A¹ est D-β-Nal.

3. Procédé de préparation d'un octapeptide de formule :
(i) H-D-Phe-Cys-Tyr-D-Trp-Lys-α-acide aminobutyrique-Cys-Thr-NH2;
(ii) H-D-β-Nal-cys-Tyr-D-Trp-Lys-α-acide aminobutyrique-Cys-Thr-NH2;
ou un de ses sels pharmaceutiquement acceptables, comprenant le couplage l'un à l'autre d'acides aminés successifs par formation de liens peptidiques.

4. Procédé de préparation d'une composition thérapeutique, le procédé comprenant le mélange d'un octapeptide, comme défini dans la revendication 1, 2 ou 3, avec un vecteur pharmaceutiquement acceptable à cet effet.

5. Procédé suivant la revendication 4, où la composition est sous la forme d'un liquide, d'une pilule, d'un comprimé ou d'une capsule, pour l'administration orale à un patient humain nécessitant le composé.

6. Procédé suivant la revendication 4, où la composition est enrobée d'une substance capable de protéger la composition contre l'acide gastrique, dans l'estomac d'un patient humain, durant une période de temps suffisante pour permettre à la composition de passer, sans être désintégrée, dans l'intestin grêle du patient humain.

7. Procédé suivant la revendication 4, où la composition est sous la forme d'une crème, d'un gel, d'un spray ou d'une pommade, pour l'application sur la peau d'un patient humain nécessitant le composé; ou la composition est sous la forme d'un liquide pouvant être administré par voie nasale, sous forme de gouttes ou de spray, à un patient humain nécessitant le composé.

8. Procédé suivant la revendication 4, où la composition est sous la forme d'un liquide pour l'administration intraveineuse, sous-cutanée, parentérale ou intrapéritonéale à un patient humain nécessitant le composé.

9. Procédé suivant la revendication 4, où la composition est sous la forme d'une composition biodégradable, à libération prolongée, pour l'administration intramusculaire à un patient humain nécessitant le composé.

10. Utilisation d'un octapeptide suivant la revendication 1, 2 ou 3 dans la préparation d'un agent de réduction de la sécrétion de l'hormone de croissance, de l'insuline, du glucagon et/ou de la sécrétion exocrine pancréatique.
